(19) **Europäisches Patentamt**
**European Patent Office**
**Office européen des brevets**

(11) Veröffentlichungsnummer: **0 001 594**
**B1**

(12)

# EUROPÄISCHE PATENTSCHRIFT

(45) Veröffentlichungstag der Patentschrift: **02.09.81**

(51) Int. Cl.³: **C 07 C 131/02**

(21) Anmeldenummer: **78101071.5**

(22) Anmeldetag: **05.10.78**

(54) **Verfahren zur Herstellung von 1,2-Dioximino-cyclohexanen.**

(30) Priorität: **24.10.77 DE 2747657**

(43) Veröffentlichungstag der Anmeldung: **02.05.79 Patentblatt 79/9**

(45) Bekanntmachung des Hinweises auf die Patenterteilung: **02.09.81 Patentblatt 81/35**

(84) Benannte Vertragsstaaten: **BE DE FR GB**

(56) Entgegenhaltungen:
**DE-A-2 441 349**
**»Organikum«**
**2. Auflage, 1963**
**VEB DEUTSCHER VERLAG DER WISSENSCHAFTEN,**
**Berlin,**
**Seiten 352, 358**

(73) Patentinhaber: **BASF Aktiengesellschaft, Carl-Bosch-Strasse 38, D-6700 Ludwigshafen (DE)**

(72) Erfinder: **Weitz, Hans-Martin, Dr., Auf dem Koeppel 40, D-6702 Bad Duerkheim 1 (DE)**
Erfinder: **Fischer, Rolf, Dr., Bergstrasse 98, D-6900 Heidelberg (DE)**

**EP 0 001 594 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von 1,2-Dioximino-cyclohexanen

Die Erfindung betrifft ein Verfahren zur Herstellung von 1,2-Dioximino-cyclohexanen durch Umsetzung von 2-Amino-cyclohexen-(2)-onen mit Hydroxylamin.

Es ist bekannt, daß man 1,2-Dioximinocyclohexan durch Umsetzung von Cyclohexan-1,2-dion (Organic Synthesis, Collective Volume IV, Seiten 229 bis 232 (1967)) oder 2-Oximinocyclohexanon (Journal of Organic Chemistry, Band 11, Seiten 771 bis 772 (1946)) mit Hydroxylamin erhält.

Es wurde nun gefunden, daß man 1,2-Dioximino-cyclohexane der Formel

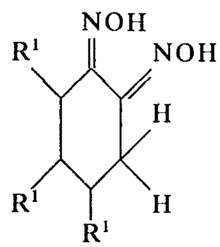

I

worin die einzelnen Reste $R^1$ gleich oder verschieden sein können und jeweils ein Wasserstoffatom, einen aliphatischen, cycloaliphatischen, araliphatischen oder aromatischen Rest bedeuten, darüber hinaus zwei benachbarte Reste $R^1$ auch zusammen mit den beiden benachbarten Kohlenstoffatomen Glieder eines Ringes bezeichnen können, vorteilhaft erhält, wenn man 2-Amino-cyclohexen-(2)-one der Formel

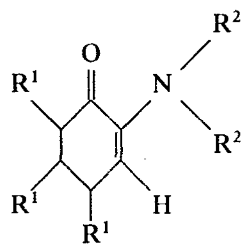

II

worin $R^1$ die vorgenannte Bedeutung besitzt, die einzelnen Reste $R^2$ gleich oder verschieden sein können und jeweils ein Wasserstoffatom, einen aliphatischen, cycloaliphatischen, araliphatischen oder aromatischen Rest bezeichnen oder auch beide Reste $R^2$ zusammen mit dem benachbarten Stickstoffatom Glieder eines heterocyclischen Ringes mit weniger oder mehr als 6 Gliedern oder die Gruppe

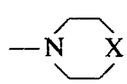

bedeuten können, X für ein Sauerstoffatom oder den Rest

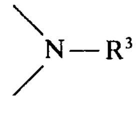

steht, $R^3$ ein Wasserstoffatom oder einen aliphatischen Rest bezeichnet, mit Hydroxylamin umsetzt.

Die Umsetzung kann für den Fall der Verwendung von 2-Morpholino-cyclohexen-(2)-on durch die folgenden Formeln wiedergegeben werden:

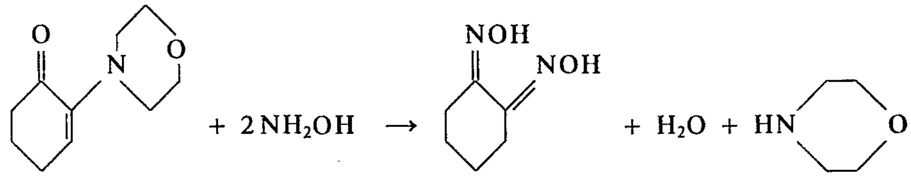

Das Verfahren nach der Erfindung liefert auf einfacherem und wirtschaftlicherem Wege, ausgehend von leichter zugänglichen und stabilen Ausgangsstoffen, 1,2-Dioximino-cyclohexane in guter Ausbeute, Reinheit sowie Raum-Zeit-Ausbeute.

Die Ausgangsstoffe II können durch Umsetzung der entsprechenden 1,2-Diketone (Zhurnal

Organicheskoi Khimii, Band 9 (1973), Seiten 2 254 bis 2 256) oder von 2,3-Epoxycycloalkanonen (Journal of Organic Chemistry, Band 35, Seiten 1 709 bis 1 711 (1970) ) mit Aminen erhalten werden. Sie werden in stöchiometrischer Menge oder im Überschuß mit Hydroxylamin, zweckmäßig in einer Menge von 2 bis 3 Mol, vorzugsweise 2,1 bis 2,5 Mol Hydroxylamin je Mol Ausgangsstoff II, umgesetzt. Bevorzugte Ausgangsstoffe II und dementsprechend bevorzugte Endstoffe I sind solche, in deren Formeln die einzelnen Reste $R^1$ und $R^2$ gleich oder verschieden sein können und jeweils ein Wasserstoffatom, einen Alkylrest mit 1 bis 6 Kohlenstoffatomen, einen Cyclohexylrest, einen Aralkylrest mit 7 bis 12 Kohlenstoffatomen, einen Phenylrest bedeuten, darüber hinaus auch zwei benachbarte Reste $R^1$ zusammen mit den beiden benachbarten Kohlenstoffatomen Glieder eines 5- oder 6gliedrigen, alicyclischen Ringes bezeichnen können, und/oder auch die beiden Reste $R^2$ zusammen mit dem benachbarten Stickstoffatom Glieder eines 5gliedrigen oder 7gliedrigen, heterocyclischen Ringes oder die Gruppe

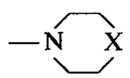

bedeuten können, X für ein Sauerstoffatom oder den Rest

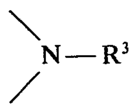

steht, $R^3$ ein Wasserstoffatom oder einen Alkylrest mit 1 bis 6 Kohlenstoffatomen bezeichnet. Die vorgenannten Reste und alicyclischen Ringe können noch durch unter den Reaktionsbedingungen inerte Gruppen, z. B. Alkylgruppen oder Alkoxygruppen mit jeweils 1 bis 4 Kohlenstoffatomen, substituiert sein.

So sind beispielsweise folgende Stoffe als Ausgangsstoffe II geeignet: In 4-Stellung, 5-Stellung oder 6-Stellung einfach oder in 4,5- bzw. 5,6- bzw. in 4,6-Stellung zweifach oder in 4,5,6-Stellung dreifach durch die Methyl-, Äthyl-, n-Propyl-, Isopropyl-, n-Butyl-, Isobutyl-, sek.-Butyl-, tert.-Butyl-, Cyclohexyl-, Benzyl-, Phenylgruppe substituierte 2-Amino-cyclohexen-(2)-one, bevorzugt unsubstituiertes 2-Amino-cyclohexen-(2)-on, entsprechend am Cyclohexenonkern substituierte oder vorzugsweise unsubstituierte Methyl-, Äthyl-, n-Propyl-, Isopropyl-, n-Butyl-, Isobutyl-, sek.-Butyl-, tert.-Butyl-, Benzyl-, Phenyl-, Cyclohexyl-, Di-(methyl)-, Di-(äthyl)-, Di-(n-propyl)-, Di-(isopropyl)-, Di-(n-butyl)-, Di-(isobutyl)-, Di-(sek.-butyl)-, Di-(tert.-butyl)-, Dibenzyl-, Diphenyl-, Dicyclohexyl-(N)-2-aminocyclohexen-(2)-one, entsprechende Aminoverbindungen mit zwei vorgenannten, aber unterschiedlichen Resten, z. B. 2-Methyläthylamino-cyclohexen-(2)-on; analoge 2-Morpholinyl-, 2-Piperazinyl-, 2-Pyrrolidinyl-, 2-(4′-Methylpiperazinyl)-, 2-Hexamethyleniminyl-(N)-cyclohexen-(2)-one.

Die Umsetzung wird im allgemeinen bei einer Temperatur von $-20$ bis $+150°C$, vorzugsweise von $+10$ bis $+90°C$, drucklos oder unter Druck, diskontinuierlich oder kontinuierlich durchgeführt. Im allgemeinen verwendet man zusätzlich unter den Reaktionsbedingungen inerte Lösungsmittel, zweckmäßig solche, in denen Hydroxylamin löslich ist. Als Lösungsmittel kommen z. B. in Frage: Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthylhexanol, Methylglykol, n-Hexanol, Isohexylalkohol, Isoheptylalkohol, n-Heptanol, 2-Äthylbutanol, Nonylalkohol, Dodecylalkohol, Methylcyclohexanol, insbesondere solche mit 1 bis 6 Kohlenstoffatomen; und entsprechende Gemische. Zweckmäßig verwendet man das Lösungsmittel in einer Menge von 200 bis 10 000 Gewichtsprozent, vorzugsweise von 400 bis 2 000 Gewichtsprozent, bezogen auf Ausgangsstoff II.

Das Hydroxylamin wird in der Regel in Form seiner Salze verwendet. Geeignete Salze sind z. B. das Chlorid, Nitrat, Sulfat, Formiat oder Acetat des Hydroxylamins. Ebenfalls ist es möglich, an Stelle der Salze Hydroxylamin selbst dem Reaktionsgemisch zuzuführen. Zweckmäßig stellt man, z. B. in der in Kirk-Othmer, Encyclopedia of Chemical Technology, Second Edition, Band 11, Seite 497 beschriebenen Arbeitsweise, eine Lösung von freiem Hydroxylamin in Wasser und/oder Alkohol her; als Alkohol verwendet man im allgemeinen die vorgenannten, insbesondere Methanol, Äthanol, n-Propanol oder n-Butanol. Man kann auch aus einem Hydroxylammoniumsalz, beispielsweise Hydroxylammoniumchlorid, Hydroxylammoniumsulfat, Hydroxylammoniumnitrat oder Hydroxylammoniumacetat, das in einem der genannten Lösungsmittel gelöst ist, mit Basen wie Natriumhydroxid, Kaliumhydroxid, Calciumhydroxid, Ammoniak, Natriumcarbonat, Natriummethylat oder Natriumäthylat Hydroxylamin freisetzen. Das anfallende Natrium-, Kalium-, Ammonium- oder Calciumsalz wird vor oder nach der Umsetzung mit Ausgangsstoff II abfiltriert.

Die Reaktion kann wie folgt durchgeführt werden: Ein Gemisch von Ausgangsstoff II, Hydroxylamin und in der Regel Lösungsmittel wird bei der Reaktionstemperatur 1 bis 20 Stunden gehalten. Der

Hydroxylaminlösung wird in der Regel unter Rühren eine Lösung des Ausgangsstoffes II in einem der genannten Lösungsmittel hinzugegeben. Es ist auch möglich, ein Gemisch aus Hydroxylammoniumsalz, Base und Ausgangsstoff II in einem der genannten Lösungsmittel ohne Herstellung einer Hydroxylaminlösung direkt umzusetzen. Der Endstoff I wird nach der Umsetzung in üblicher Weise, z. B. durch Filtration, gegebenenfalls nach Fällung, beispielsweise mit Wasser, und gegebenenfalls Umkristallisation, abgetrennt.

Die nach dem Verfahren der Erfindung herstellbaren Verbindungen I stellen Komplexbildner für Schwermetallionen dar. Sie sind wertvolle Ausgangsstoffe für die Herstellung von Diaminen, z. B. durch Reduktion der Oximinogruppen mit katalytisch aktiviertem Wasserstoff, Lithiumaluminiumhydrid oder Zink (Houben-Weyl, Methoden der Organischen Chemie, Band 11, Teil 1, Seiten 495 bis 502). Derartige Diamine können als alkalische Katalysatoren, Zusätze zu Epoxidharzen und als Ausgangsstoffe für die Synthese von Farbstoffen und Arzneimitteln verwendet werden.

Die in den folgenden Beispielen angeführten Teile bedeuten Gewichtsteile.

## Beispiel 1

14 Teile Kaliumhydroxid in 32 Teilen Methanol werden mit einer Lösung (50°C) von 17,4 Teilen Hydroxylammoniumhydrochlorid in 71 Teilen Methanol versetzt. Zu der Hydroxylamin-Lösung gibt man bei 20°C innerhalb von 15 Minuten 18,1 Teile 2-Morpholinyl-(N)-2-cyclohexen-1-on, gelöst in 40 Teilen Methanol. Das Reaktionsgemisch wird nun 4 Stunden zum Rückfluß erhitzt, dann auf 22°C abgekühlt. Nach dem Absaugen des angefallenen Kaliumchlorids wird das Filtrat am Rotationsverdampfer bei 65°C Badtemperatur eingedampft. Der kristalline Rückstand wird mit 20 Teilen Eiswasser digeriert und abgesaugt. Nach dem Trocknen im Vakuum werden 12,5 Teile 1,2-Dioximinocyclohexan (87,9% der Theorie) erhalten. Schmelzpunkt 185 bis 187°C (aus $H_2O$).

## Beispiel 2

14 Teile Kaliumhydroxid in 32 Teilen Methanol werden mit einer Lösung (50°C) von 17,4 Teilen Hydroxylammoniumhydrochlorid in 71 Teilen Methanol versetzt. Zu der Hydroxylamin-Lösung gibt man bei 20°C innerhalb von 15 Minuten 19,4 Teile N-Methylpiperazinyl-(N)-2-cylohexen-1-on, gelöst in 40 Teilen Methanol. Das Reaktionsgemisch wird nun 16 Stunden bei +4°C gerührt, dann auf 22°C erwärmt. Nach dem Absaugen des angefallenen Kaliumchlorids wird das Filtrat am Rotationsverdampfer bei 65°C Badtemperatur eingedampft. Der kristalline Rückstand wird mit 20 Teilen Eiswasser digeriert und abgesaugt. Nach dem Trocknen im Vakuum werden 7,9 Teile 1,2-Dioximinocyclohexan (55,6% der Theorie) erhalten. Schmelzpunkt 186 bis 188°C (aus $H_2O$).

## Beispiel 3

14 Teile Kaliumhydroxid in 32 Teilen Methanol werden mit einer Lösung (50°C) von 17,4 Teilen Hydroxylammoniumhydrochlorid in 71 Teilen Methanol versetzt. Zu der Hydroxylamin-Lösung gibt man bei 20°C innerhalb von 15 Minuten 18,7 Teile Anilino-(N)-2-cyclohexen-1-on, gelöst in 40 Teilen Methanol. Das Reaktionsgemisch wird nun 16 Stunden bei 22°C gerührt, dann auf 4°C abgekühlt. Nach dem Absaugen des angefallenen Kaliumchlorids wird das Filtrat am Rotationsverdampfer bei 65°C Badtemperatur eingedampft. Der kristalline Rückstand wird mit 20 Teilen Eiswasser digeriert und abgesaugt. Nach dem Trocknen im Vakuum werden 12,9 Teile 1,2-Dioximinocyclohexan (90,7% der Theorie) erhalten. Schmelzpunkt 185 bis 188°C (aus $H_2O$).

## Beispiel 4

14 Teile Kaliumhydroxid in 79 Teilen Äthanol werden mit einer Lösung (50°C) von 17,4 Teilen Hydroxylammoniumhydrochlorid in 198 Teilen Äthanol versetzt. Zu der Hydroxylamin-Lösung gibt man bei 20°C innerhalb von 15 Minuten 16,7 Teile Diäthylamino-(N)-2-cyclohexen-1-on, gelöst in 40 Teilen Äthanol. Das Reaktionsgemisch wird nun 4 Stunden am Rückfluß erhitzt, dann auf 20°C abgekühlt. Nach dem Absaugen des angefallenen Kaliumchlorid wird das Filtrat am Rotationsverdampfer bei 65°C Badtemperatur eingedampft. Der kristalline Rückstand wird mit 20 Teilen Eiswasser digeriert und abgesaugt. Nach dem Trocknen im Vakuum werden 9,4 Teile 1,2-Dioximinocyclohexan (66,1% der Theorie) erhalten. Schmelzpunkt 186 bis 188°C (aus $H_2O$).

Beispiel 5

14 Teile Kaliumhydroxid in 32 Teilen Methanol werden mit einer Lösung (50°C) von 17,4 Teilen Hydroxylammoniumhydrochlorid in 71 Teilen Methanol versetzt. Zu der Hydroxylamin-Lösung gibt man bei 20°C innerhalb von 15 Minuten 16,5 Teile Pyrrolidino-(N)-2-cyclohexen-1-on, gelöst in 40 Teilen Methanol. Das Reaktionsgemisch wird nun 4 Stunden zum Rückfluß erhitzt, dann auf 20°C abgekühlt. Nach dem Absaugen des angefallenen Kaliumchlorids wird das Filtrat am Rotationsverdampfer bei 65°C Badtemperatur eingedampft. Der kristalline Rückstand wird mit 20 Teilen Eiswasser digeriert und abgesaugt. Nach dem Trocknen im Vakuum werden 11,3 Teile 1,2-Dioximinocyclohexan (79,5% der Theorie) erhalten. Schmelzpunkt 184 bis 187°C (aus $H_2O$).

**Patentanspruch**

Verfahren zur Herstellung von 1,2-Dioximinocyclohexanen der Formel

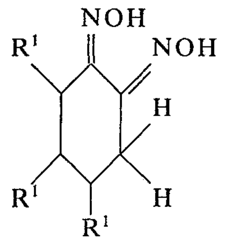

I

worin die einzelnen Reste $R^1$ gleich oder verschieden sein können und jeweils ein Wasserstoffatom, einen aliphatischen, cycloaliphatischen, araliphatischen oder aromatischen Rest bedeuten, darüber hinaus zwei benachbarte Reste $R^1$ auch zusammen mit den beiden benachbarten Kohlenstoffatomen Glieder eines Ringes bezeichnen können, dadurch gekennzeichnet, daß man 2-Amino-cyclohexen-(2)-one der Formel

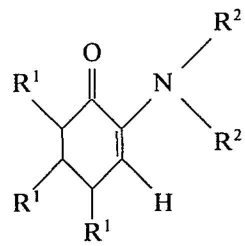

II

worin $R^1$ die vorgenannte Bedeutung besitzt, die einzelnen Reste $R^2$ gleich oder verschieden sein können und jeweils ein Wasserstoffatom, einen aliphatischen, cycloaliphatischen, araliphatischen oder aromatischen Rest bezeichnen oder auch beide Reste $R^2$ zusammen mit dem benachbarten Stickstoffatom Glieder eines heterocyclischen Ringes mit weniger oder mehr als 6 Gliedern oder die Gruppe

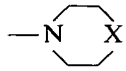

bedeuten können, X für ein Sauerstoffatom oder den Rest

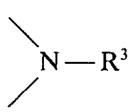

steht, $R^3$ ein Wasserstoffatom oder einen aliphatischen Rest bezeichnet, mit Hydroxylamin umsetzt.

**Claim**

A process for the production of a 1,2-dioximinocyclohexane of the formula

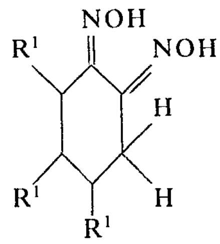

I

where the individual radicals $R^1$ may be identical or different and each denotes hydrogen or an aliphatic, cycloaliphatic, araliphatic or aromatic radical, and in addition two adjacent radicals $R^1$ together with the two adjacent carbon atoms may denote members of a ring, characterized in that a 2-aminocyclohexen-(2)-one of the formula

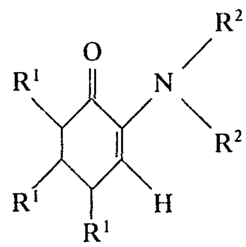

II

where $R^1$ has the above meaning, the individual radicals $R^2$ may be identical or different and each denotes hydrogen or an aliphatic, cycloaliphatic, araliphatic or aromatic radical, or the two radicals $R^2$ together with the adjacent nitrogen atom may also denote members of a heterocyclic ring with less or more than 6 members, or the group

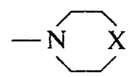

X denoting oxygen or the radical

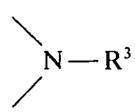

where $R^3$ denotes hydrogen or an aliphatic radical, is reacted with hydroxylamine.

**Revendication**

Procédé de préparation des 1,2-dioximino-cyclohexanes de formule

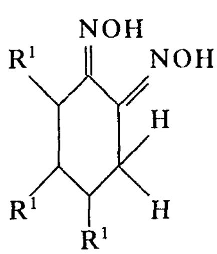

I

dans laquelle les divers symboles $R^1$, ayant des significations identiques ou différentes, représentent chacun un atome d'hydrogène, un reste aliphatique, cycloaliphatique, araliphatique ou aromatique, deux symboles $R^1$ voisins pouvant également représenter ensemble, avec les deux atomes de carbone

voisins, des chaînons d'un cycle, caractérisé en ce que l'on fait réagir avec l'hydroxylamine les 2-amino-cyclohexène-(2)-ones de formule

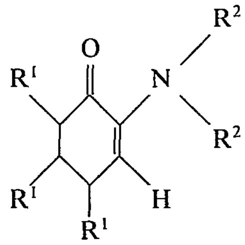

II

dans laquelle $R^1$ a les significations indiquées ci-dessus, les divers symboles $R^2$, identiques ou différents, représentent chacun un atome d'hydrogène, un reste aliphatique, cycloaliphatique, araliphatique ou aromatique ou bien encore les deux symboles $R^2$ forment ensemble et avec l'atome d'azote voisin des chaînons d'un hétérocycle contenant moins ou plus de 6 chaînons ou le groupe

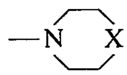

X représente un atome d'hydrogène ou le reste

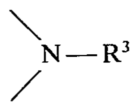

$R^3$ représente un atome d'hydrogène ou un reste aliphatique.